# EUROPEAN PATENT SPECIFICATION

(11) **EP 3 231 838 B1**
(45) Date of publication and mention of the grant of the patent: **28.08.2019**
(21) Application number: 15867518.1
(22) Date of filing: 29.05.2015
(51) Int. Cl.: C08L 5/04, C08L 5/08, C08L 89/00, C08K 5/544, C08K 5/5419, C08K 5/5415, C08K 3/36, C08J 3/24, G02B 1/00, G02C 7/04, A61F 9/007

(54) **HIGH OXYGEN PERMEABILITY MATERIAL BASED ON MARINE BIOLOGICAL SUBSTANCES AND PREPARATION METHOD AND USE THEREOF**
MATERIAL MIT HOHER SAUERSTOFFDURCHLÄSSIGKEIT AUF DER BASIS VON BIOLOGISCHEN MEERESSUBSTANZEN SOWIE HERSTELLUNGSVERFAHREN UND VERWENDUNG DAVON
MATÉRIAU À HAUTE PERMÉABILITÉ À L'OXYGÈNE À BASE DE SUBSTANCES BIOLOGIQUES MARINES ET SON PROCÉDÉ DE PRÉPARATION ET SON UTILISATION

(30) Priority: 12.12.2014 CN 201410766381
(43) Date of publication of application: 18.10.2017
(73) Proprietor: Qingdao Chunghao Tissue Engineering Co. Ltd., Qingdao, Shandong 266101 (CN)
(72) Inventor: WANG, Baoquan, Anqiu Shandong 262100 (CN); LI, Qing, Qingdao Shandong 266061 (CN)
(74) Representative: Nederlandsch Octrooibureau
(86) International application number: PCT/CN2015/080218
(87) International publication number: WO 2016/090863

(56) References cited:
- WO-A1-2010/076923
- CN-A- 101 987 887
- CN-A- 102 378 783
- CN-A- 102 558 599
- CN-A- 102 558 599
- CN-A- 104 448 407
- US-A- 4 365 050
- US-A- 5 910 518

## Description

### Field of the Invention

The present invention belongs to the field of medical equipment, in particular, involves a material with high oxygen permeability based on marine biological substance, preparation method and use thereof.

### Background of the Invention

Corneal contact lens (hereinafter referred to as contact lens) is a kind of delicate ophthalmic medical instrument, the main purpose is to correct vision, keep natural appearance, medical treatment, et al. The oxygen permeability coefficient (DK) or oxygen transfer coefficient (DK/t) is commonly used to state the oxygen permeation ability of corneal contact lens, wherein D is the diffusion coefficient of O₂ in the material, K is the dissolving coefficient of O₂ in the material, DK is the product of D and K, the unit of which is barrer, and t is the center thickness of the lens, the unit of which is millimetres (mm). Researcher suggests that, to meet the day wear requirements of no corneal edema, DK/t value must be greater than 24 barrers/mm. When wear overnight, DK/t value must be greater than 87 barrers/mm, but corneal edema still occur in a rate of 4%. Only when DK/t > 125 barrers/mm, it can ensure that extended wear will not result in relative symptoms caused by a lack of oxygen. Wearers can wear contact lens for consecutive seven days, 30 days or longer, which can reduce the trouble caused by wearing for everyday and provide the possibility for the treatment of slow release and controlled release of drugs etc. in the eye. The oxygen permeation ability is the primary factor to determine that whether the lens is suitable for daily wear or extended wear, and the majority of consumers will consider that whether the lens is suitable for extended wear, when they choose contact lens. Therefore, cornea contact lens with high oxygen permeability has a large market demand. Now, DK/t value of the common hydrogel contact lens remains to be improved.

Hydrogel contact lenses have been described in prior art documents WO2010/076923A1. This document discloses a hydrogel contact lens comprising an acryl monomer, an oligosaccharide and a silicone compound.

CN101987887A reports on the preparation of PDMS (polydimethylsiloxane) silicone rubber modified by chitosan and hyaluronic acid.

Furthermore, CN102558599Areports on the synthesis of a silicic acid/calcium alginate hybrid material with a mesoporous silica gel layer on the surface.

### Summary of the Invention

The purpose of the present invention is to overcome poor oxygen permeability and other defects in the existing corneal contact lens, and provide a material with high oxygen permeability based on marine biological substance, preparation method and use thereof.

In order to obtain corneal contact lens with high oxygen permeability and good comfort, the inventors of the present invention did a lot of experiments. It was found that cornea contact lens which is prepared by using marine biological substances such as alginic acid and silicon-containing substances with silicon-oxy groups as raw materials showed better oxygen permeability and comfort. Therefore, in the first aspect, the present invention provides a material with high oxygen permeability which is obtained by compounding a silicon-containing substance with silicon-oxy group and a bioactive substance selected from one or more of alginic acid, collagen, hyaluronic acid and salts thereof, in the presence of a crosslinking agent, wherein high oxygen permeability is defined as an oxygen transfer coefficient (DK/t) value of at least 81 barrers/mm, as measured according to Chinese national standard GB/T11417.7-2012, and wherein based on 100 parts by weight of the bioactive substance in the material with high oxygen permeability, the amount of silicon-containing substance is 1-10 parts by weight.

In the second aspect, the present invention provides a preparation method of the material with high oxygen permeability according to the first aspect, which includes: the silicon-containing substance and bioactive substance are mixed under acidic condition, then the mixture is subjected to crosslinking reaction in the presence of a crosslinking agent.

The preferable method of the present invention includes the following steps:
(1) a silicon-containing substance dispersed in an acid solution containing 10⁻³-10⁻⁶mol/L of hydrogen ion and a bioactive substance are mixed, in which the silicon-containing substance is selected from at least one of silica gel, 3-aminopropyl trimethoxysilane, tetraethoxy-silicone and diethoxydimethylsilane, the particle size of silica gel is preferably 200-800 meshes, the bioactive substance is selected from at least one of alginic acid, sodium alginate, potassium alginate, collagen, hyaluronic acid, sodium hyaluronate and potassium hyaluronate.
(2) the mixture obtained in step (1) is subjected to crosslinking reaction for 2-5 h at 15-35°C in the presence of a crosslinking agent;
wherein based on 100 parts by weight of the bioactive substance, the amount of silicon-containing substance is 1-10 parts by weight and the amount of crosslinking agent is 0.01-5 parts by weight.

In the third aspect, the present invention provides a material with high oxygen permeability prepared by the method according to the second aspect.

In the fourth aspect, the present invention provides use of the material with high oxygen permeability according to the first aspect or third aspect, or the method according to the second aspect in preparation of corneal contact lens, corneal scaffold material or corneal substitute.

Through the above technical solution, the present invention obtained a material with high oxygen permeability that can satisfy the requirement of high oxygen permeability for extended wear and show good wearing comfort (high water content). It has important practical significance and broad application prospects for the development of long-term wearing corneal contact lens and slow and controlled release of therapeutic preparation for corneal disease. Furthermore, the employing of marine biological substance (alginic acid, collagen, hyaluronic acid and their salts) with good biocompatibility makes the material with high oxygen permeability have good biocompatibility and high safety performance.

Other characteristics and advantages of the present invention will be described in details in the subsequent embodiments.

### Detailed Description of the Embodiments

Below the embodiments of the present invention are described.

The material with high oxygen permeability provided in the present invention is obtained by compounding a silicon-containing substance with silicon-oxy group and a bioactive substance selected from one or more of alginic acid, collagen, hyaluronic acid, and salts thereof, in the presence of a crosslinking agent, in the presence of a crosslinking agent, wherein high oxygen permeability is defined as an oxygen transfer coefficient (DK/t) value of at least 81 barrers/mm, as measured according to Chinese national standard GB/T11417.7-2012, and wherein based on 100 parts by weight of the bioactive substance in the material with high oxygen permeability, the amount of silicon-containing substance is 1-10 parts by weight. Crosslinking is present among at least part of (or all of) the bioactive substances.

According to the present invention, the collagen can be various common collagen which is derived from mammals etc. Preferably, the collagen is derived from fish. More preferably, the collagen has a molecular weight of 8-350kDa. Most preferably, the collagen is type I collagen.

According to the present invention, the molecular weight of hyaluronic acid is preferably 80-2000kDa.

In the present invention, the salt of alginic acid, collagen or hyaluronic acid can also be used to obtain the material with high oxygen permeability in the present invention. Preferably, the bioactive substance is selected from at least one of alginic acid, sodium alginate, potassium alginate, collagen, hyaluronic acid, sodium hyaluronate and potassium hyaluronate.

According to the present invention, the silicon-containing substance can be the silicon-containing substance with silica group commonly used for the preparation of corneal contact lens in the field. Preferably, the silicon-containing substance is selected from at least one of silica gel, 3-aminopropyl trimethoxysilane, tetraethoxy-silicone and diethoxydimethylsilane. The particle size of silica gel is preferably 200-800 meshes.

Based on 100 parts by weight of the bioactive substance, the amount of crosslinking agent is preferably 0.01-5 parts by weight, more preferably 0.1-3 parts by weight. The crosslinking agent may be any commonly used matter making the bioactive substance crosslinked in the field. Preferably, the crosslinking agent is CaCl₂, and/or a mixture of 1-ethyl-3-(3-dimethylaminopropyl)-carbodiimide and N-hydroxysuccinimide (EDC/NHS, the molar ratio of EDC/NHS is usually 1: 0.1-2).

The method for preparing the material with high oxygen permeability in the present invention includes: the silicon-containing substance and bioactive substance are mixed under acidic condition, and the mixture is subjected to crosslinking reaction in the presence of a crosslinking agent.

According to the present invention, the amount of silicon-containing substance is 1-10 parts by weight, based on 100 parts by weight of bioactive substance.

According to the present invention, the collagen can be various common collagen which is derived from mammals etc. Preferably, the collagen is derived from fish. More preferably, the collagen has a molecular weight of 8-350kDa. Most preferably, the collagen is type I collagen. In order to maintain the activity of collagen, when the material with high oxygen permeability is obtained by compounding a collagen and a silicon-containing substance, silicon-containing substance and bioactive substance are mixed at 0-20°C.

According to the present invention, the molecular weight of hyaluronic acid is preferably 80-2000kDa.

In the present invention, the salt of alginic acid, collagen or hyaluronic acid can also be used to obtain the material with high oxygen permeability in the present invention. Preferably, the bioactive substance is selected from at least one of alginic acid, sodium alginate, potassium alginate, collagen, hyaluronic acid, sodium hyaluronate and potassium hyaluronate.

According to the present invention, the silicon-containing substance may be the silicon-containing substance with silica group commonly used for the preparation of corneal contact lens in the field. Preferably, the silicon-containing substance is selected from at least one of silica gel, 3-aminopropyl trimethoxysilane, tetraethoxy-silicone and diethoxydimethylsilane. The particle size of silica gel is preferably 200-800 meshes.

According to the present invention, based on 100 parts by weight of the bioactive substance, the amount of crosslinking agent is preferably 0.01-5 parts by weight, more preferably 0.1-3 parts by weight. The crosslinking agent may be any commonly used matter making the bioactive substance crosslinked in the field. Preferably, the crosslinking agent is CaCl₂, and/or a mixture of 1-ethyl-3-(3-dimethylaminopropyl)-carbodiimide and N-hydroxysuccinimide (EDC/NHS, the molar ratio of EDC/NHS is usually 1: 0.1-2). The crosslinking agent may be used in form of solution, and the concentration of crosslinking agent in the solution may be 0.05-3mol /L.

Preferably, the acidic condition manifests pH 3-6.

According to the preferred embodiment of the present invention, before mixing the silicon-containing substance and bioactive substance, the silicon-containing substance is dispersed in an acid solution containing 1O⁻³-10⁻⁶mol/L of hydrogen ion. Per mole (gram) silicon-containing substance is generally dispersed in 0.2-10L (or 0.2-5L) acid solution. Dispersing may be carried out in a conventional manner, as long as the silicon-containing substance is dispersed fully, thus facilitate the subsequent step of crosslinking. For example, ultrasonic dispersion may be adopted (the frequency for ultrasonic dispersion may be 35-50 kHz, the time for ultrasonic dispersion may be 15-30min). When the silicon-containing substance is silica gel, it may be purified in order to obtain a material with high oxygen permeability manifesting better comfort and light-admitting quality. The methods of purification may be any method known in the field. More preferably, the method further includes: before being dispersed in an acid solution, the silicon-containing substance is treated as follows (to purify the silica gel):
(a) the silicon-containing substance is dealt with acid liquid for 2-10h at 50-100°C, and then washed with water until become neutral;
(b) the silicon-containing substance processed by step (a) is immersed in a mixed solution of hydrochloric acid solution and ethanol for 2-10h at 20-50°C, and then washed with water until become neutral.

In step (a), the concentration of hydrogen ion in the acid liquid is preferably 1-10mol/L. The preferable acid contained in the acid liquid is at least one of sulfuric acid, nitric acid and hydrochloric acid.

In the mixture of hydrochloric acid solution and ethanol metioned in step (b), the concentration of hydrogen ion in the hydrochloric acid solution is 0.005-0.05mol/L, and the volume ratio of hydrochloric acid solution and ethanol is 1: 0.1-5.

In the present invention, the acid contained in the acid solution is at least one of acetic acid, sulfuric acid, nitric acid and hydrochloric acid. The acid contained in the acid solution, the acid contained in the acid liquid used in step (a) and the acid used in step (b) may be the same or different.

According to the present invention, there's no special requirements for the condition for crosslinking reaction, as long as the bioactive substance is crosslinked. Preferably, the condition for crosslinking reaction includes: a temperature of 15-35°C and a time of 2-5h.

According to the present invention, the method may further include: before crosslinking reaction, bubbles are removed from the mixture. The method of removing bubbles may be centrifugation or vacuum-pumping, which can be selected by the skilled in the art, and and need not be repeated here.

According to a preferred embodiment of the present invention, the method for preparing the material with high oxygen permeability includes the following steps:
(1) a silicon-containing substance dispersed in an acid solution containing 10⁻³-10⁻⁶mol/L of hydrogen ion and a bioactive substance are mixed, in which the silicon-containing substance is selected from at least one of silica gel, 3-aminopropyl trimethoxysilane, tetraethoxy-silicone and diethoxydimethylsilane, the particle size of silica gel is 200-800 meshes, the bioactive substance is selected from at least one of alginic acid, sodium alginate, potassium alginate, collagen, hyaluronic acid, sodium hyaluronate and potassium hyaluronate.
(2) the mixture obtained in step (1) is subjected to crosslinking reaction for 2-5 h at 15-35°C in the presence of a crosslinking agent;
wherein based on 100 parts by weight of the bioactive substance, the amount of silicon-containing substance is 1-10 parts by weight and the amount of crosslinking agent is 0.01-5 parts by weight.

In the present invention, the bioactive substance may be mixed with the silicon-containing substance in the form of solution, and the concentration of bioactive substance in the solution may be 5-200mg/mL, for example, the concentration of alginic acid or salts thereof may be 10-200mg/mL; the concentration of collagen or salts thereof may be 5-50mg/mL; the concentration of hyaluronic acid or salts thereof may be 10-40mg/mL.

The silicon-containing substance and bioactive substance used in the present invention can be obtained by chemical synthesis methods or may be commercially available and need not be repeated here.

The present invention also provides the material with high oxygen permeability obtained by the above-mentioned method.

Furthermore, the present invention also provides use of the above-mentioned method or the material with high oxygen permeability in preparation of corneal contact lenses, corneal scaffold material or corneal substitute.

When the corneal contact lenses, corneal scaffold material or corneal substitute is prepared by the above-mentioned method of the present invention, the crosslinking reaction may be carried out in a mould (for corneal contact lenses, corneal scaffold material or corneal substitute) directly. For example, the mixture of silicon-containing substance and bioactive substance is placed in a mould directly, and then the crosslinking agent is added into the mould so as to initiate crosslinking reaction, thus the corneal contact lenses, corneal scaffold material or corneal substitute with specific shape may be obtained.

Below the present invention will be described in details by referring to examples.

In the following examples, the silica gel is purchased from Qingdao Ocean Chemical Co. Ltd; 3-aminopropyl trimethoxysilane is purchased from Sigma; Sodium alginate is purchased from Qingdao Bright Moon Seaweed Limited; fish collagen is isolated from deep-sea cod with molecular weight of 300-350kDa (i.e. type I collagen); hyaluronic acid is purchased from Solarbio with molecular weight of 80-2000kDa; EDC (No. 39391) and NHS (No. 130672) are purchased from Sigma; the thickness of the corneal contact lens is measured using corneal pachymeter.

### Example 1

(1) The silica gel (300-400 meshes) was treated with concentrated sulfuric acid (98 wt%) for 2h at 70°C and washed several times with distilled water until become neutral, and then treated with a mixture of 0.01M hydrochloric acid solution and absolute ethanol (v/v=1:1) for 2h at 37°C and washed several times with distilled water until become neutral, then dried at 20°C for 24h in reserve.
(2) 0.01g silica gel treated by step (1) was dispersed in 2mL acetic acid solution (pH 4) ultrasonically (40 kHz) for 20min and then added into 25mg/mL of sodium alginate solution (4mL), vortex mixing thoroughly, removing the bubbles by centrifugation. 300µL mixed solution was dropped into the contact lens mould, then the contact lens mould was plugged using a plug slowly. 10µL 0.2mol/L of CaCl₂ solution was dropped from the gap of the edge followed with gently rotating the plug. The contact lens mould was placed for 2h at 25°C and a corneal contact lens with thickness of 100µm was obtained.

### Example 2

(1) The silica gel (800 meshes) was treated with concentrated sulfuric acid (98 wt%) for 5h at 80°C and washed several times with distilled water until become neutral, and then treated with a mixture of 0.01M hydrochloric acid solution and absolute ethanol (v/v=1:5) for 2h at 50°C and washed several times with distilled water until become neutral, then dried at 20°C for 24h in reserve.
(2) 0.008g silica gel treated by step (1) was dispersed in 2mL acetic acid solution (pH 6) ultrasonically (40 kHz) for 20min and then added into 20mg/mL of fish collagen solution (5mL), vortex mixing thoroughly, removing the bubbles by centrifugation. 300µL mixed solution was dropped into the contact lens mould, then the contact lens mould was plugged using a plug slowly. 10µL 0.05mol/L of a mixed solution of EDC/NHS was dropped from the gap of the edge followed with gently rotating the plug. The contact lens mould was placed for 4h at 20°C and a corneal contact lens with thickness of 100µm was obtained.

### Example 3

(1) 1mg of 3-aminopropyl trimethoxysilane (ultrasonically dispersed (40 kHz) for 20min) was dispersed into 3mL of acetic acid solution (pH 3) at 4°C, and then added into 10mL of sodium hyaluronate solution (10mg/mL) and vortex mixing thoroughly in icewater bath for 10min, removing the bubbles by centrifugation. 100µL mixed solution was dropped into the contact lens mould, then the contact lens mould was plugged using a plug slowly. 10µL 1mol/L of a mixed solution of EDC/NHS was dropped from the gap of the edge followed with gently rotating the plug. The contact lens mould was placed for 5h at 15°C and a corneal contact lens with thickness of 100µm was obtained.

### Example 4

Corneal contact lens was prepared according to the method of Example 2, the difference is using the"pig collagen" (with molecular weight of 8-10kDa) instead of "fish collagen", and a corneal contact lens with thickness of 100µm was obtained.

### Test Example 1

Various parameters of corneal contact lenses prepared according to the above methods were measured, such as light transmission rate, oxygen transfer coefficient (DK/t), water content and so on. The specific methods refer to the national standard (GB/T11417.5-2012 and GB/T11417.7-2012), and the results were shown in Table 1.

**Table 1**

| Example No. | Light transmission rate (%) | DK/t (barrers/mm) | Water content (wt%) |
|---|---|---|---|
| Example 1 | 95 | 106 | 63 |
| Example 2 | 93 | 129 | 70 |
| Example 3 | 91 | 102 | 52 |
| Example 4 | 87 | 81 | 47 |

As can be seen from the results as shown in Table 1, the material with high oxygen permeability obtained according to the examples had high light transmission rate and oxygen transfer coefficient which satisfied the requirements of an ideal extended wear, and had high water content. Thus, the material with high oxygen permeability of the present invention did have high oxygen permeability, high water content so as to manifest better comfort.

## Claims

1. A material with high oxygen permeability which is obtained by compounding a silicon-containing substance with silicon-oxy group and a bioactive substance selected from one or more of alginic acid, collagen, hyaluronic acid, and salts thereof, in the presence of a crosslinking agent, wherein high oxygen permeability is defined as an oxygen transfer coefficient (DK/t) value of at least 81 barrers/mm, as measured according to Chinese national standard GB/T11417.7-2012, and wherein based on 100 parts by weight of the bioactive substance in the material with high oxygen permeability, the amount of silicon-containing substance is 1-10 parts by weight.

2. The material with high oxygen permeability according to claim 1, wherein the silicon-containing substance is selected from at least one of silica gel, 3-aminopropyl trimethoxysilane, tetraethoxy-silicone and diethoxydimethylsilane.

3. The material with high oxygen permeability according to claim 1 or 2, wherein based on 100 parts by weight of the bioactive substance, the amount of crosslinking agent is 0.01-5 parts by weight.

4. The material with high oxygen permeability according to any of claims 1-3, wherein the crosslinking agent is calcium chloride, and/or a mixture of 1-ethyl-3-(3-dimethyl aminopropyl) carbodiimide and N-hydroxysuccinimide.

5. A method for preparing the material with high oxygen permeability according to any of claims 1 to 4, including: the silicon-containing substance and bioactive substance are mixed under acidic condition, and then the mixture is subjected to crosslinking reaction in the presence of a crosslinking agent.

6. The method according to claim 5, wherein the acidic condition includes pH 3-6.

7. The method according to claim 5 or 6, wherein the silicon-containing substance is silica gel and the method further includes: before being dispersed in an acid solution, the silicon-containing substance is treated as follows:
(a) the silicon-containing substance is dealt with acid liquid for 2-10h at 50-100°C, and then washed with water until become neutral;
(b) the silicon-containing substance processed by step (a) is immersed in a mixed solution of hydrochloric acid solution and ethanol for 2-10h at 20-50 °C, and then washed with water until become neutral.

8. The method according to claim 7, wherein the acid contained in the acid solution is at least one of acetic acid, sulfuric acid, nitric acid and hydrochloric acid.

9. The method according to claim 5, wherein the condition for crosslinking reaction includes: a temperature of 15 - 35°C and a time of 2-5 h.

10. A method for preparing the material with high oxygen permeability according to claim 1, including the following steps:
(1) a silicon-containing substance dispersed in an acid solution containing 10⁻³-10⁻⁶mol/L of hydrogen ion and a bioactive substance are mixed, in which the silicon-containing substance is selected from at least one of silica gel, 3-aminopropyl trimethoxysilane, tetraethoxy-silicone and diethoxydimethylsilane, the bioactive substance is selected from at least one of alginic acid, sodium alginate, potassium alginate, collagen, hyaluronic acid, sodium hyaluronate and potassium hyaluronate;
(2) the mixture obtained in step (1) is subjected to crosslinking reaction for 2-5 h at 15-35°C in the presence of a crosslinking agent;
wherein based on 100 parts by weight of the bioactive substance, the amount of silicon-containing substance is 1-10 parts by weight and the amount of crosslinking agent is 0.01-5 parts by weight.

11. A material with high oxygen permeability prepared by the method according to any of claims 5-10.

12. Use of the method according to any of claims 5-10, or the material with high oxygen permeability according to any of claims 1-4 and 11 in preparation of corneal contact lens, corneal scaffold material or corneal substitute.

## Patentansprüche

1. Ein Material mit hoher Sauerstoffdurchlässigkeit, welches durch Vermischen einer siliciumhaltigen Substanz mit einer Silicium-Oxygruppe und einer bioaktiven Substanz, ausgewählt aus einer oder mehreren von Alginsäure, Kollagen, Hyaluronsäure und Salzen dieser, in Anwesenheit eines Vernetzers erhalten wird, wobei hohe Sauerstoffdurchlässigkeit als ein Sauerstofftransferkoeffizient (DK/t) mit einem Wert von mindestens 81 barrer/mm, gemessen gemäß dem chinesischen nationalen Standard GB/T11417.7-2012, definiert ist, und wobei der Gehalt an siliciumhaltiger Substanz, basierend auf 100 Gewichtsanteilen der bioaktiven Substanz in dem Material mit hoher Sauerstoffdurchlässigkeit, 1-10 Gewichtsanteile beträgt.

2. Das Material mit hoher Sauerstoffdurchlässigkeit nach Anspruch 1, wobei die siliciumhaltige Substanz ausgewählt ist aus zumindest einem von Silikagel, 3-Aminopropyltrimethoxysilan, Tetraethoxysilikon und Diethoxydimethylsilan.

3. Das Material mit hoher Sauerstoffdurchlässigkeit nach Anspruch 1 oder 2, wobei der Gehalt an Vernetzer basierend auf 100 Gewichtsanteilen der bioaktiven Substanz 0.01-5 Gewichtsanteile beträgt.

4. Das Material mit hoher Sauerstoffdurchlässigkeit nach einem der Ansprüche 1-3, wobei der Vernetzer Calciumchlorid und/oder eine Mischung aus 1-Ethyl-3-(3-dimethylaminopropyl)carbodiimid und N-Hydroxysuccinimid ist.

5. Ein Verfahren zum Herstellen des Materials mit hoher Sauerstoffdurchlässigkeit nach einem der Ansprüche 1 bis 4, einschließend: die siliciumhaltige Substanz und die bioaktive Substanz werden unter saurer Bedingung gemischt und dann wird die Mischung in Anwesenheit eines Vernetzers einer Vernetzungsreaktion unterzogen.

6. Das Verfahren nach Anspruch 5, wobei die saure Bedingung einen pH von 3-6 einschließt.

7. Das Verfahren nach Anspruch 5 oder 6, wobei die siliciumhaltige Substanz Silikagel ist und das Verfahren weiterhin einschließt: bevor die siliciumhaltige Substanz in einer sauren Lösung dispergiert wird, wird sie wie folgt behandelt:
(a) die siliciumhaltige Substanz wird für 2-10h bei 50-100°C mit saurer Flüssigkeit behandelt und anschließend mit Wasser bis zur Neutralität gewaschen;
(b) die in Schritt (a) aufbereitete siliciumhaltige Substanz wird für 2-10h bei 20-50°C in eine gemischte Lösung aus Salzsäure und Ethanol getaucht und anschließend mit Wasser bis zur Neutralität gewaschen.

8. Das Verfahren nach Anspruch 7, wobei die in der sauren Lösung enthaltene Säure zumindest eine von Essigsäure, Schwefelsäure, Salpetersäure und Salzsäure ist.

9. Das Verfahren nach Anspruch 5, wobei die Bedingung für die Vernetzungsreaktion einschließt: eine Temperatur von 15-35°C und eine Zeit von 2-5h.

10. Ein Verfahren zum Herstellen des Materials mit hoher Sauerstoffdurchlässigkeit nach Anspruch 1, das die folgenden Schritte einschließt:
(1) eine in einer sauren Lösung, die 10⁻³-10⁻⁶ mol/L Wasserstoffionen enthält, dispergierte siliciumhaltige Substanz und eine bioaktive Substanz werden gemischt, wobei die siliciumhaltige Substanz ausgewählt ist aus zumindest einem von Silikagel, 3-Aminopropyltrimethoxysilan, Tetraethoxysilikon und Diethoxydimethylsilan, die bioaktive Substanz ausgewählt ist aus zumindest einem von Alginsäure, Natriumalginat, Kaliumalginat, Kollagen, Hyaluronsäure, Natriumhyaluronat und Kaliumhyaluronat;
(2) die in Schritt (1) erhaltene Mischung wird in Anwesenheit eines Vernetzers für 2-5h bei 15-35°C einer Vernetzungsreaktion unterzogen;
wobei der Gehalt an siliciumhaltiger Substanz basierend auf 100 Gewichtsanteilen der bioaktiven Substanz 1-10 Gewichtsanteile beträgt und der Gehalt an Vernetzer 0.01-5 Gewichtsanteile beträgt.

11. Ein Material mit hoher Sauerstoffdurchlässigkeit hergestellt durch das Verfahren nach einem der Ansprüche 5-10.

12. Verwendung des Verfahrens nach einem der Ansprüche 5-10 oder des Materials mit hoher Sauerstoffdurchlässigkeit nach einem der Ansprüche 1-4 und 11 in der Herstellung einer Kornealkontaktlinse, von Hornhautgerüstmaterial oder Hornhautersatz.

## Revendications

1. Matériau à forte perméabilité à l'oxygène, obtenu en composant une substance contenant du silicium avec un groupe silicium-oxy et une substance bioactive choisie parmi un ou plusieurs de l'acide alginique, du collagène, de l'acide hyaluronique et de leurs sels, en présence d'un agent de réticulation, dans lequel la forte perméabilité à l'oxygène est définie comme une valeur du coefficient de transfert d'oxygène (DK/t) d'au moins 81 barrers/mm, mesurée conformément à la norme nationale chinoise GB/T11417.7-2012 et dans lequel, par rapport à 100 parties en poids de la substance bioactive dans le matériau à forte perméabilité à l'oxygène, la quantité de substance contenant du silicium est comprise entre 1 et 10 parties en poids.

2. Matériau à forte perméabilité à l'oxygène selon la revendication 1, dans lequel la substance contenant du silicium est choisie parmi au moins l'un du gel de silice, du 3-aminopropyl triméthoxysilane, du tétraéthoxysilicone et du diéthoxydiméthylsilane.

3. Matériau à forte perméabilité à l'oxygène selon la revendication 1 ou 2, dans lequel, par rapport à 100 parties en poids de la substance bioactive, la quantité d'agent de réticulation est comprise entre 0,01 et 5 parties en poids.

4. Matériau à forte perméabilité à l'oxygène selon l'une quelconque des revendications 1 à 3, dans lequel l'agent de réticulation est du chlorure de calcium et/ou un mélange de 1-éthyl-3-(3-diméthyl aminopropyl) carbodiimide et de N-hydroxysuccinimide.

5. Procédé de préparation du matériau à forte perméabilité à l'oxygène selon l'une quelconque des revendications 1 à 4, comprenant: la substance contenant du silicium et la substance bioactive sont mélangées dans des conditions acides, puis le mélange est soumis à une réaction de réticulation en présence d'un agent de réticulation.

6. Procédé selon la revendication 5, dans lequel les conditions acides comprennent un pH compris entre 3 et 6.

7. Procédé selon la revendication 5 ou 6, dans lequel la substance contenant du silicium est un gel de silice, le procédé comprenant en outre l'étape suivante : avant d'être dispersée dans une solution acide, la substance contenant du silicium est traitée de la façon suivante :
(a) la substance contenant du silicium est traitée par un liquide acide pendant 2 à 10 h entre 50 et 100°C, puis lavée à l'eau jusqu'à devenir neutre ;
(b) la substance contenant du silicium traitée à l'étape (a) est immergée dans une solution mélangée de solution d'acide chlorhydrique et d'éthanol pendant 2 à 10 h entre 20 et 50°C, puis lavée à l'eau jusqu'à devenir neutre.

8. Procédé selon la revendication 7, dans lequel l'acide contenu dans la solution acide est au moins l'un de l'acide acétique, de l'acide sulfurique, de l'acide nitrique et de l'acide chlorhydrique.

9. Procédé selon la revendication 5, dans lequel les conditions pour la réaction de réticulation comprennent : une température comprise entre 15 et 35°C et une durée de 2 à 5 h.

10. Procédé de préparation du matériau à forte perméabilité à l'oxygène selon la revendication 1, comprenant les étapes suivantes :
(1) une substance contenant du silicium dispersée dans une solution acide contenant entre 10⁻³ et 10⁻⁶ mol/L d'ions hydrogène et une substance bioactive sont mélangées, dans laquelle la substance contenant du silicium est choisie parmi au moins l'un du gel de silice, du 3-aminopropyl triméthoxysilane, du tétraéthoxysilicone et du diéthoxydiméthylsilane, la substance bioactive est choisie parmi au moins l'un de l'acide alginique, de l'alginate de sodium, de l'alginate de potassium, du collagène, de l'acide hyaluronique, de l'hyaluronate de sodium et de l'hyaluronate de potassium ;
(2) le mélange obtenu à l'étape (1) est soumis à une réaction de réticulation pendant 2 à 5 h entre 15 et 35°C en présence d'un agent de réticulation ;
dans lequel, par rapport à 100 parties en poids de la substance bioactive, la quantité de substance contenant du silicium est de 1 à 10 parties en poids et la quantité d'agent de réticulation est comprise entre 0,01 et 5 parties en poids.

11. Matériau à forte perméabilité en oxygène préparé par le procédé selon l'une quelconque des revendications 5 à 10.

12. Utilisation du procédé selon l'une quelconque des revendications 5 à 10 ou du matériau à forte perméabilité en oxygène selon l'une quelconque des revendications 1 à 4 et 11, en préparation d'une lentille de contact cornéenne, d'un matériau d'échafaudage cornéen ou d'un substitut cornéen.
